(19) Europäisches Patentamt European Patent Office Office européen des brevets

(11) **EP 2 462 236 B1**

(12) **EUROPEAN PATENT SPECIFICATION**

(45) Date of publication and mention of the grant of the patent:
**09.03.2016 Bulletin 2016/10**

(51) Int Cl.:
***C12P 19/34*** *(2006.01)*

(21) Application number: **10800660.2**

(86) International application number:
**PCT/US2010/042426**

(22) Date of filing: **19.07.2010**

(87) International publication number:
**WO 2011/009117 (20.01.2011 Gazette 2011/03)**

(54) **DETECTION OF SHORT RNA SEQUENCES**

NACHWEIS KURZER RNA-SEQUENZEN

DÉTECTION DE COURTES SÉQUENCES D ARN

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **17.07.2009 US 226451 P**

(43) Date of publication of application:
**13.06.2012 Bulletin 2012/24**

(73) Proprietor: **Brown University Providence, RI 02912 (US)**

(72) Inventors:
• **TRIPATHI, Anubhav Providence, RI (US)**
• **SARMA, Aartik Providence, RI (US)**
• **ONG, Carmichael Providence, RI (US)**

(74) Representative: **Sonn & Partner Patentanwälte Riemergasse 14 1010 Wien (AT)**

(56) References cited:
**WO-A2-2005/092038**    **US-A1- 2009 181 389**

• **WEUSTEN ET AL.: 'Principles of quantitation of viral loads using nucleic acid sequence-based amplification in combination with homogenous detection using molecular beacons.' NAR vol. 30, no. 6, 2002, pages 1 - 7, XP002436126**

**Description**

**Field of the Invention**

Disclosed is an assay for detection of short sequences of RNA in a synthetic or clinically isolated sample. Particular reference is made to detecting RNA based pathogens, focusing on H5 influenza.

**Background of the Invention**

A current assay used widely is Nucleic Acid Sequence-Based Amplification (NASBA). A drawback to the NASBA technique is the secondary structure of target molecules. These can either hinder or completely stop an amplification reaction.

A major problem in medicine is creating a sensitive, point of care device for RNA pathogens. Faster and more efficient diagnosis would lead to more efficient treatment of patients. Polymerase chain reaction (PCR) assays require precise equipment that is not conducive to a point of care solution. Nucleic Acid Sequence-Based Amplification (NASBA) may currently be the best choice for a point of care device as it involves using an isothermal amplification step lasting only 90 minutes. NASBA, however, has previously not worked as expected in our laboratory, and it may be due to secondary structure in the RNA that prevents efficient primer binding and enzyme progression.

*Diagnosis of Influenza A H5N1*

Many techniques have been utilized in order to diagnose influenza. Historically, there arc two ways to confirm the presence of the virus: detection of a person's immune response to the virus or detection of the virus itself. Four techniques that cover antibody detection, or the immune response, are virus neutralization test (NT), hemagglutination inhibition (HI), enzyme immunoassay (EIA), and complement fixation. These tests check for influenza antibodies in an individual. These antibodies have their peak levels occur between four to seven weeks after infection. Thus, these techniques are not widely used for clinical applications but can be important in analyzing and diagnosing retrospectively. These techniques, especially virus NT and HI assays, have been utilized to identify subtypes of influenza. Despite the high specificity of virus NT and HI assays, these techniques are extremely labor intensive and take at least some weeks before results are reached. Thus, there would be many problems to overcome if one were to try to utilize viral antibody detection [33].

Detection of the virus could be considered a more important approach for clinically relevant applications. There are three general approaches in this branch of detection: immunospecific assays for viral antigen detection, viral isolation, and nucleic acid testing. The first group, immunospecific assays, encompasses two categories: rapid antigen tests and immunofluorescence microscopy [33].

Rapid antigen tests provide a very quick result and currently serve as one option for point of care detection. Commercial kits, such as Directigen Flu A and QuickVue influenza test, are already on the market and ready to use [33]. Some kits are reported to detect subtype H5N1 virus [34]. These tests use specimens such as nasopharyngeal aspirates, nasopharyngeal swabs, and throat swabs. Many factors have an impact upon the sensitivity of the test. Reports suggest that sensitivity is useful at about two days after symptoms appear when viral shedding is maximal [33]. These tests, however, can have a wide range of specificity and sensitivity. The range of reported sensitivity is between 39% and 100%, and the range for specificity is between 51 % and 100%, varying with the kit as well as from where the sample was obtained. This varied range of sensitivity coupled with the lack of ability to subtype the different HA groups are the main drawbacks of this technique [35].

Immunofluorescence microscopy, which includes direct fluorescent antibody tests (DFA) and immunofluorescent antibody tests (IFA), works by placing respiratory epithelial cells onto a slide and adding a series of specific antibodies. The slide is then viewed via fluorescence microscopy. These tests can give a result within about four hours. There are, however, some drawbacks in comparison to rapid antigen tests as a fluorescence microscope is needed, and a trained technician must carry out the test in order to perform the experiment as well as interpret the results. Despite the drawbacks, its higher sensitivity than rapid antigen testing and ability to subtype make this technique a valuable asset to influenza diagnosis [33].

Viral isolation is used as it has a very high sensitivity level, down to about 10 pfu/mL (plaque forming units). Thus, the sensitivity for this assay is greater than the rapid antigen tests. Furthermore, it allows for laboratories to increase their stocks of virus for further studies [37]. In addition, viral culture continues to be an important method in providing critical information about circulating strains and subtypes of influenza. In conventional test culture, a patient's sample material is added to a cell culture. Then, the culture is monitored for signs of cytopathic effect. This alone, however, does not confirm that the culture is infected by influenza as the effect can be due to a number of viruses. Confirmation is typically performed via antibody staining and analyzing the culture with a fluorescence microscope. Disadvantages of this strategy include the length of time to receive a result, which can take up to 14 days, the need for a specialized technician, the requirement of live, viable virus, and the need for highly certified laboratories (BSL-3) in cases where one is dealing with highly pathogenic strains of influenza [33].

It has been reported that low-speed centrifugation increases the viral infectivity of cells. It is thought that this step disrupts cells and allows foreign viruses to enter more efficiently. In turn, this enhances the sensitivity of the culture, decreasing the time required for a diagnosis to between 18 and 48 hours. This technique may cause the virus to become nonviable. Thus, passaging cells becomes an issue, especially when a lab is utilizing cell culture to increase viral RNA for nucleic acid techniques.

A test directly detecting viruses is nucleic acid testing (NAT). In general, NAT works by specifically amplifying DNA or RNA or both in the presence of a specific sequence of nucleic acid. For influenza, typically amplification occurs in the presence of viral influenza RNA. NAT is considered more sensitive and specific than virus isolation, and in some cases it has replaced viral isolation as a reference standard. In addition, because nucleic acid is targeted and amplified, both viable and nonviable virus can be used in an assay. These techniques also give investigators information about not only the subtype but also allows for sequence analysis that can be done after amplification. Results can be obtained within four to six hours. Two methods within this category that are used and researched widely are reverse transcription polymerase chain reaction (RT-PCR) and nucleic acid sequence based amplification (NASBA) [33].

PCR is a cyclic process consisting of three steps: denaturing, annealing, and extending. Denaturing the DNA involves separating the two strands. This usually involves heating the sample to 95°C. Heat denaturation of nucleic acids is reversible, unlike other methods such as chemical denaturation. The denaturation step allows the two primers to anneal to the DNA. Primers are short, single-stranded sequences of DNA that are reverse complementary to the DNA strands to be amplified. This step occurs at a lower temperature, which varies depending on the DNA and primers, in order to allow for annealing to occur [39]. The last step, extension of primers, utilizes the thermostable DNA polymerase *Thermus aquaticus*, also known as *Taq* polymerase. *Taq* polymerase extends the primers in a 5' to 3' direction. This step occurs at about 72°C, which is a higher temperature than the annealing step but a lower temperature than the denaturing step. The thermostable property of *Taq* is extremely important as it allows for the cyclic nature of the reaction to take place without needing to add reagents [40]. A typical cycle length is between three and five minutes, with a total of 20 to 40 cycles, thus the total length for PCR is usually a little over three hours [39].

A noted variation of PCR utilized for detecting RNA sequences is RT-PCR. Reverse transcription is an extra step needed before the PCR cycle because the starting material is viral RNA rather than DNA. Generally, an ssDNA primer hybridizes to a specific section of RNA and then is extended by an enzyme, such as avian myeloblastosis virus reverse transcriptase (AMV-RT) or Moloney murine leukemia virus reverse transcriptase (MMLV-RT). Another variation to RT-PCR that has been developed is multiplex PCR. In these assays, multiple primer sets are used either for detecting multiple genes of a single pathogen or subtype or for detecting multiple subtypes of influenza at the same time [41]. The former has been shown for H5N1 in a multiplex RT-PCR that, in a single tube, can detect the genes coding for M, H5, and N1. This could become useful in surveillance as mutations could be noted in a current strain [42]. The latter type of multiplex RT-PCR has been shown to be able to differentiate between H1, H3, and H5; N1 and N2; and virus types A and B [43].

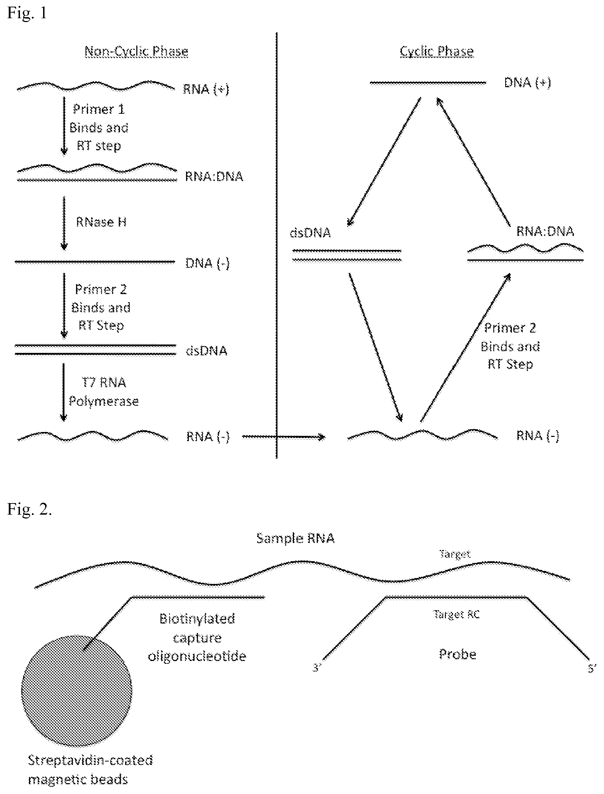

Another method for amplification is NASBA. It is reported that NASBA will exponentially amplify targets without temperature cycling, between 37°C and 41 °C. Thus, less sophisticated equipment is necessary [44, 45]. NASBA also generally requires fewer cycles than PCR to achieve the same amplification, which reduces the length of the reaction to between 1.5 and 2 hours [46]. Finally, NASBA has been utilized in detection of human papilloma virus (HPV) [47], HIV-1 [48], and influenza A virus of all HA subtypes [49]. Typically, NASBA requires three enzymes (a reverse transcriptase, an RNA polymerase that catalyzes the formation of RNA in the 5'→ 3' direction, and a non-specific endonuclease and catalyzes the cleavage of RNA. Examples of each are AMV-RT (also e.g., Moloney murine leukemia virus (MMLV-RT) and HIV-RT), T7 RNA polymerase (also, *e.g.,* T3, and SP6 polymerases), and RNase H. NASBA also requires nucleoside triphosphates (both dNTPs and rNTPs), two DNA primers, and the correct buffer conditions. A diagram of the reaction is in Fig. 1. The first part runs non-cyclically. The target RNA, RNA (+), binds to primer 1, and AMV-RT extends the primer. It is important to note that primer 1 contains a promoter region for T7 RNA polymerase. RNAse H, which selectively degrades RNA in RNA-DNA hybrids, degrades the RNA, leaving the extended DNA, DNA (-). Primer 2 binds to the DNA and AMV-RT extends this primer, creating a double stranded DNA (dsDNA). T7 RNA polymerase then creates many copies of the negative RNA strand, RNA (-), from the dsDNA template. This triggers the cyclic phase. Primer 2 then binds RNA (-) and is extended by AMV-RT. RNAse H degrades the RNA in the RNA-DNA hybrid, leaving DNA (+). Primer I then binds to DNA (+) and AMV-RT extends the primer, creating dsDNA. T7 RNA polymerase completes the cycle by transcribing more RNA (-). Thus, the negative strand, RNA (-), is exponentially amplified. [46]. Noted also is locked nucleic acid (as well as other analogues such as TNA, GNA, and PNA). Locked nucleic acid (LNA), often referred to as inaccessible RNA, is a modified RNA nucleotide. The ribose moiety of an LNA nucleotide is modified with an extra bridge connecting the 2' oxygen and 4' carbon. The bridge "locks" the ribose in the 3'-endo (North) conformation, which is often found in the A-form of DNA or RNA. These nucleotides LNA, TNA, GNA, and PNA are collectively termed "Other Nucleotides."

Like PCR, there have been variations on NASBA developed to make this procedure more flexible. There have been some successful attempts at multiplex NASBA, where multiple NASBA primers are used in the same reaction. One utilizes the method to amplify enteric viruses [50] while another has been used to detect hepatitis A virus and rotavirus simultaneously [51]. A protocol has also been reported that amplifies DNA using NASBA. Because the strands of DNA must be separated first, an initial denaturing step at 95°C is needed.

Fluorescence in detection is utilized with molecular beacons [54]. Molecular beacons are single stranded DNA designed to be reverse complementary to itself on its two ends. This creates a hairpin shape with a stem region and a loop region. The ends are modified such that one has a fluorescent tag and the other has a fluorescent quencher. Thus, when the hairpin is closed, no fluorescence is emitted. The loop section of the DNA strand is reverse complementary to the target and is longer than the stem section. In the presence of the target nucleic acid, the beacon will anneal to the target, and the two ends of the beacon will move away from each other. This leads to fluorescence that can be quantified via a fluorometer. Real-time detection is a feature of this technique. [55].

**Summary of the Invention**

The method described is an extension of the Nucleic Acid Sequence Based Amplification (NASBA) protocol. The method disclosed is useful to exponentially amplify strands of RNA, complementary to at least a portion of a Target nucleotide sequence (i.e., RNA, DNA, and Other Nucelotides) if the Target nucleotide sequence is present in a sample. "Sample" is used in reference to an aliquot, suspension, or fraction that contains the nucleotide (e.g., RNA or DNA) under investigation.

The modified protocol in one embodiment employing ligation of contiguous probe fragments relies on four short DNA oligonucleotide sequences. The four sequences are:

ProbeLeft "5' - TargetRCLeft - HybridSeqRC -3'",
ProbeRight "5' - Primer2-TargetRCRight - 3'",
Primer1 "5' - T7 - HybridSeq - 3'"', and
Primer2.

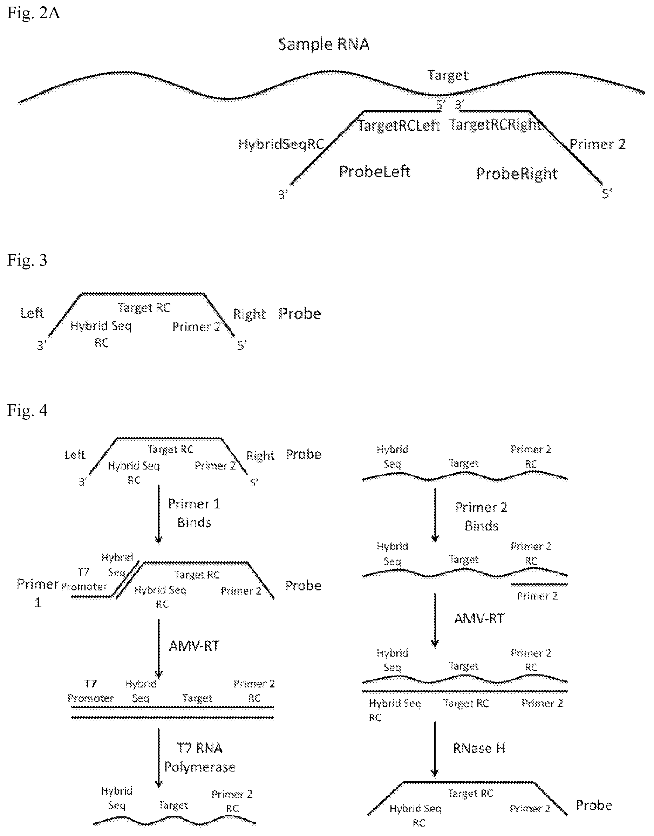

Fig. 2A shows a diagrammatic view of TargetRCRight and TargetRCLeft as hybridized to a sample RNA. Left and Right as used herein with reference to target sequences shall be understood to mean Left as to a nucleotide or nucleotide sequence positioned closer to the 5' end, while Right is used to indicate a nucleotide or nucleotide sequence positioned closer to the 3' end.

TargetRCLeft and TargetRCRight are short sequences that are reverse complementary (RC) to adjacent sequences in a sample being assayed. HybridSeq and Primer2 are designed to minimize the secondary structure of the RNA produced in this reaction, while maintaining a strong binding energy to their reverse complements.

The step of washing away Probe can be avoided with an additional reaction. ProbeLeft and ProbeRight are single-stranded DNA sequences designed such that when the 5'-end of ProbeRight is ligated to the 3'-end of ProbeL, the completed ligated sequence forms a sequence identical to the Probe sequence that would be used in the reaction. ProbeLeft and ProbeRight can be placed in a reaction mix containing a DNA ligase that ligates the fragments if and only if they are adjacent to each other while hybridized to a complementary DNA or RNA sequence. (Fig. 2A) To facilitate this juxtaposition, both ProbeLeft and ProbeRight usefully contain sequences that are reverse complements of the sequence being detected. Examples of ligases include *E. coli* DNA ligase, *Taq* DNA ligase, and T4 DNA ligase. For clarity, hybridizing conditions are conditions that permit reannealing of nucleotides with their complementary bases.

ProbeLeft and ProbeRight are usefully added to a reaction mix containing the reagents described herein along with a ligase, as the ligation step may be carried out in the solution conditions described above and optimally at about the functional temperature for the ligase. A reaction time of about 10-90 minutes at such temperature (e.g., about 16-65°C, depending on the ligase). As a caution, it is noted that various enzymes are heat inactivated (e.g., T7 polymerase at about 41 °C.) It will be understood if the reaction mix is heated above the inactivation point these heat labile enzymes should be added after the ligation step. Alternatively, Sample and ProbeLeft and ProbeRight can be reacted under the optimal reaction conditions for the ligase in a separate tube or container, and some of this reaction mix can be placed in place of the Sample in the previously described reaction conditions.

In one embodiment, the oligonucleotides are added to a Tris-buffered pH 8.3 solution containing MgCl2, dithiothreitol, nucleoside triphosphates, deoxynucleoside triphosphates, DMSO, and isolated RNA. The solution is heated to 65°C for 5 minutes to disrupt the RNA secondary structure, and then cooled to 16°C. Once the solution has cooled, an enzyme mix containing AMV reverse trancriptase (AMV-RT), T7 RNA polymerase (T7Pol), RNaseH, and T4 DNA Ligase is added to the solution, and the solution is held at 16°C for 10 minutes. T4 DNA ligase forms a phosphodiester bond between adjacent DNA fragments that are hybridized to DNA or RNA strands. This ligates ProbeLeft and ProbeRight if, and only if, the Target sequence is present to make one combined oligonucleotide ("5' - Primer2-TargetRC-Hybrid-SeqRC - 3'", "Probe"). The sequence is then exponentially amplified at 41°C in the following cycle of reactions. RNAseH selectively degrades RNA hybridzed to DNA, so the Probe sequence is separated from the original RNA, which is destroyed. Primer 1 hybridizes with Probe (HybridSeq +HybridSeqRC). AMV-RT reads primed single stranded RNA and DNA sequences to synthesize DNA in the 5'->3' direction, and creates a double stranded DNA with one strand with the sequence 5,-T7-HybridSeq-TargetRC-Primer2RC-3'. The T7 region of the sequence is the highly conserved T7 polymerase promoter sequence. This acts as a recognition site for the T7 polymerase to begin in vitro transcription of an RNA sequence. Transcription is believed to occur by reading the DNA strand that the T7 promoter is hybridized to and transcribing the RNA sequence complementary to the DNA strand. In the case of double-stranded DNA templates, this means the RNA strand has the same sequence as the original top strand of DNA. The polymerase does not copy its own promoter sequence, so the polymerase will make the following RNA strand "5'- HybridSeq-TargetRC-Primer2RC-3'". Since the transcription is not primer-dependent, the polymerase makes multiple copies of the RNA for each template that is made. The RNA sequences are templates for reverse transcription by AMV-RT after priming by Primer2, which makes a DNA strand with the sequence 5' - Primer2-Probe-HybridSeqRC-3', which is the Probe sequence. This exponentially amplifies RNA as a growing number of dsDNA templates are created. The progression of this reaction is monitored with molecular beacons, which are short DNA sequences that form a hairpin structure. A fluorophore and a fluorescence quencher are on the ends of the beacon sequence. As a result, fluorescence is not observable when the hairpin is in its closed state, but is observed when the sequence is open. The loop of the sequence is complementary to a target RNA or DNA sequence, while the 5'-end of the sequence is complementary to the 3'-end. The sequence is designed so that the binding energy of the loop to its complement is slightly greater than the binding energy of the stem to itself. This creates a very specific and sensitive reporter for the complement to the loop sequence. In this reaction, the beacon is designed to detect the presence of Primer2RC. Primer2RC is produced when the reaction is successful. A non-sequence specific method of assaying RNA synthesis (like RiboGreen) may also be used to evaluate the progression of the reaction.

This protocol detects multiple target sequences (such as different genotypes associated with the same clinically relevant phenotype) with the same molecular beacon by varying the Target sequence while keeping the Primer2 and HybridSeq sequences unchanged. This is believed to provide more favorable amplification thermodynamics than typical multiplex NASBA reactions, which are less efficient due to the number of primers present. In addition, the use of a single beacon lowers costs and simplifies detection.

This invention comprises A method of amplify Target RNA comprising the steps of introducing at least one Target RNA (*e.g.,* H5 influenza) to a sample containing probe nucleotide under hybridizing conditions;
wherein said Target RNA comprises three regions,
said first region being a Hybrid Seq RC region; and,
a second region, being a Target RC regions contiguous with said first region; and
a third region being a Primer 2 region contiguous with said second region; and, selectively amplifying the RNA of said Target RNA, and optionally detecting and/or quantifying the amplified RNA. In some embodiments detection and/or quantification is by gel electrophoresis or by fluorescence. Particularly noted is detection and/or quantification using molecular beacons. Multiple molecular beacons are useful to detect and/or quantify multiple Target RNAs.

In one embodiment of the method the amplifying of step (ii) comprises transcribing hybridized Target RNA into double-stranded DNA. The instant method also usefully employs a primer containing a T7 promoter sequence which binds to the probe RNA. The instant method further employs transcribing it by means of a reverse transcriptase such as AMV-RT. AMV-RT is useful to transcribe a reverse complementery DNA sequence which creates a DNA-RNA hybrid. Noted is an embodiment including transcribing said resulting double stranded DNA into RNA. One noted method of transcribing is by means of an RNA polymerase that catalyzes the formation of RNA in the 5'→ 3' direction. An aspect of the method further includes only the RNA strand of a DNA-RNA hybrid being degraded, optionally by RNase H. The resulting DNA strand is then primed with a primer at the 3' end and the transcription process is repeated with AMV-RT. Transcription produces a double stranded DNA (a template) from which T7 RNA polymerase transcribes an RNA.

In the claimed method the Hybrid Seq RC region, Target RC region and said Primer 2 region each comprise from about 8 to about 35 bases which number as to each region may be the same or different. In addition and optionally within the method a molecular beacon may be hybridized to the amplified RNA.

The invention further includes a method of detecting target nucleotide comprising

(i) exposing ProbeLeft nucleotides and ProbeRight nucleotides under hybridizing conditions to a Probe-specific ligase;
(ii) permitting ligating of said ProbeLeft and ProbeRight nucleotides if and only if they are adjacent to each other while said ProbeLeft and ProbeRight nucleotides are hybridized to a complementary nucleotide sequence; and,
(iii) detecting and/or quantifying the presence or absence of said ligated ProbeLeft with ProbeRight nucleotide.

## Brief Description of the Drawings

Fig. 1. Fig. 1 is a schematic of both the non-cylic and cyclic phases of the NASBA reaction. Wavy lines represent RNA and straight lines represent DNA.

Fig. 2. Fig. 2 is a schematic of the capture step to the SMART assay.

Fig. 2A shows a diagrammatic view of TargetRCRight and Target RCLeft as hybridized to a sample RNA.

Fig. 3. Fig. 3. is a schematic of the SMART Probe with the variable arms on either side of the center portion.

Fig. 4. Fig. 4 is a schematic of the amplification step to the SMART assay.

Fig. 5. Fig 5 is a diagram showing where the molecular beacon binds in the SMART assay for real-time detection.

Fig. 6. Fig. 6 is a picture of a gel from Gel electrophoresis of the initial testing of the amplification step.

Fig. 7. Fig. 7 is a gel from an experiment varying DMSO concentration. Four conditions of different concentrations of probe, 100 nM, 10 nM, 1 nM, and 0 nM, were run for each DMSO concentration, 5% and 15%. The concentrations of probe go from left to right with each DMSO condition group.

Fig. 8. is a gel plot of an experiment varying Tris-HCl pH. Four conditions of different concentrations of probe, 100 nM, 10 nM, 1 nM, and 0 nM, were run for each pH condition, 8.0 and 8.3. The concentrations of probe go from left to right with each pH group.

Fig. 9 is a gel plot of electrophoresis of various nucleic acids in the SMART Assay using the Small RNA Assay.

Fig. 10. Fig. 10 is an electropherogram plot of a mix of Primers and Probe, cDNA, and cDNA with T7 RNA polymerase.

Fig. 11 is an electropherogram plot of three reactions. The first reaction (light gray)shows a reaction with AMV-RT only. The second line (dark gray) shows the reaction with AMV-RT and T7. The final line (black) represents the reaction involving all three enzymes.

Fig. 12. is a plot of relative fluorescence versus time for many dilutions of the Probe from Set 1.

Fig 13 is a design of a chip for SMART assay utilizing electrophoresis detection. Red areas denote a heating implement for a constant temperature.

## Detailed Description of the Invention

The assay presented here, coined a Simple Method to Amplify RNA Targets (SMART), involves amplifying a probe engineered for improved binding to primers as well as minimizing the secondary structure of nucleic acids to be amplified. In some embodiments, this technique utilizes isothermal, cyclic amplification. These parameters are useful in point of care settings as such conditions minimize the equipment needs. In particular embodiments, the disclosed assay is employed in a microfluidic chip platform.

The work presented here particularly notes H5 influenza as a target. In addition and without limitation, the method is useful with any RNA based pathogen.

In particular embodiments, the SMART assay binds engineered ssDNA probes to an RNA target. Then the engineered probes are selectively amplified rather than the target RNA itself being amplified as is typical in the NASBA protocol. Selectively amplifying shall be understood to mean that at least about 80% or more and preferably 90% or more (by base count) of the RNA synthesized in this cycle of reactions is Target RNA and contiguous regions (*i.e.,* first region and third region) or fragments thereof.

One advantage of these probes is that they are short and can be engineered to have minimal secondary structure. This is distinct from the sample nucleotide. Detection of the amplified nucleic acid is usefully performed by any method but note is made of two standard methods: gel electrophoresis or fluorescence via molecular beacons.

This invention will be better understood with resort to the following definitions:

A. "Short" as to RNA sequences, shall mean from about 8 to about 35 bases (also termed nucleotides or nt). with particular reference to about 10 or about 18 to 25 nucleotides. This term is particularly directed to the TargetRC portion of the SMART probe.

B. SMART probe is an oligonucleotide as shown diagrammatically in Fig. 3. having variable arms on either side of the center portion ("Target RC"). The variable arms vary from about 5, or about 10 or about 18 to 25 or 35 nucleotides each. One said variable arm is termed Hybrid Seq. RC and one is termed Primer 2. As to such variable arms, it is understood and contemplated that the sequences used for hybridizing Primer 1 or Primer 2 to the Probe sequence may include part of the sequence being tested for in the Probe sequence.

C. Hybrid Seq. RC shall mean a sequence on the 3' end of the Probe sequence that is sufficiently complementary to the 3' end of Primer 1 to hybridize under reaction conditions.

D. Primer 1 shall mean a primer comprising a 5' promoter region for an RNA polymerase that catalyzes the formation of RNA in the 5'→ 3' direction (e.g., T7 RNA polymerase) and a HybridSeq on the 3' end that hybridizes to the 3' end of the Probe sequence to enable the creation of a double-stranded DNA sequence by a DNA-dependent DNA polymerase (e.g., AMV RT) to be used as template for a DNA-dependent RNA polymerase.

E. Primer 2 shall mean an oligonucleotide sequence complementary to the 3' end of RNA transcribed from the DNA template synthesized in "D", which will hybridize to this sequence to permit the transcription of a DNA:RNA hybrid catalyzed by an RNA-dependent DNA polymerase (such as AMV-RT).

F. Target RC shall mean a short oligonucleotide sequence that is reverse complementary (RC) to a sequence on a Target RNA sequence.

G. ProbeLeft and ProbeRight shall mean single-stranded DNA sequences designed such that when the 5'-end of ProbeRight is ligated to the 3'-end of ProbeLeft, the completed ligated sequence forms a sequence identical to the Probe sequence that would be used in the reaction. The point of ligation shall be within the region described as TargetRC It is noted that in some embodiments of the instant invention the step of using magnetic-streptavidin coated beads bound to biotinylated capture probes a useful first step where some method of hybridizing the probes to the target RNA and washing away the unhybridized probes is required. Beads are one such method. Other useful methods include capturing the target RNA on another surface, like a microfluidic channel.

Two SMART assay sets for H5 influenza were tested. Set 1 was more efficient than Set 2. Set 1 had more favorable Primer 1 dimer formation (-8.5 kcal/mol for Set 1 vs. -4.2 kcal/mol for Set 2) while Set 2 had more favorable RNA self-binding (-4.2 kcal/mol for Set 1 vs. -5.2 kcal/mol for Set 2). Without being bound by any particular theory, it is believed that this indicates that secondary structure of binding sites has a greater effect on reaction efficiency than primer-primer interactions. In addition, it appeared that the optimal Tris-HCl buffer pH was about 8.0. This is lower than many literature sources for NASBA. Similarly, it appeared that optimal DMSO concentration was about 15% v/v.

Using gel electrophoresis, it was confirmed that the expected products were produced. Molecular beacons were shown to be a viable option for detection at concentrations in the femtomolar range.

The SMART assay is an improvement over nucleic acid methods for detection and permits a point of care device. NASBA is isothermal and typically runs at a relatively low temperature (41°C) and reportedly down to about 37°C. Also noted are available heat-stable equivalents for all the enzymes in the reaction which function up to about 70°C. Indeed, in some embodiments, exact temperatures are not necessary to NASBA as compared to PCR [44, 45]. In addition, the amplification step for NASBA lasts for 90 minutes. Experiments have shown that it is difficult to find NASBA primers that yield a good result. Without being bound by any particular theory it is believed that the complex conformation that an RNA strand can take, especially in a sequence as long as influenza which is approximately 1700 bases, makes it difficult for primers to consistently bind at some places.

The characteristics of a short amplification time and an isothermal reaction are useful for a point of care device. To overcome the problem of NASBA assays that are ineffective, the instant method amplifies an engineered probe rather than the RNA target itself. This probe binds to the RNA targets, if present, and will be substantially washed away otherwise.

In a specific embodiment, magnetic, streptavidin-coated beads are bound to biotinylated capture probes via the streptavidin-biotin bond. The capture probe is reverse complementary to the RNA target in one region. The engineered NASBA probe is reverse complementary to another region, which is selected to be a more favorable binding site. With a series of washes, the unbound probe is substantially washed away and thus will have limited amplification. The separation step is presented in Fig. 2.

In Fig. 2, RNA is shown by a wavy line, and DNA is shown by a straight line. Streptavidin-coated magnetic beads are added to a solution containing biotinylated capture oligonucleotides, or capture probes. These capture probes are reverse complementary to the target RNA strand.

In a specific embodiment of the invention , the engineered NASBA type probe has two, 25 nucleotide long arms on each end with a middle section that is the reverse complementary part to the RNA of interest. The middle section, which can vary from about 5, or about 10 or about 18 to 25 or 35 nucleotides due to the specificity and binding efficiency desired, is small in comparison to the two, variable arms. The arms can also vary about 5, or about 10 or about 18 to 25 or 35 nucleotides each. Engineering the length takes into account three concepts. If the segment is overlong it may fold onto itself thus slowing down reactions. Its design also reflects a length to have binding energy sufficient to stay bound through various washing steps, but not so long so as to unduly degrade binding specificity.

Thus, arm length is selected to improve/permit sufficient amplification speed. They are optimized for efficient binding in order to push the reaction forward. Furthermore, they are optimized to reduce its own secondary structure, which addresses one the concerns about traditional NASBA methods. A schematic is shown as Fig. **3**.

In Fig. 3, one variable end has the same sequence as Primer 2. The other variable end is called "Hybrid Seq RC" as it is reverse complementary to a segment of Primer 1. Useful aspects of these regions are further delineated in the amplification steps.

The design of the amplification step is similar to the NASBA reaction. However, in distinction, the starting target is an engineered probe strand rather than the positive strand RNA. In some instances, the same enzymes as NASBA are used. AMV-RT extends DNA primers that are hybridized to either DNA or RNA segments. T7 RNA polymerase transcribes double stranded DNA downstream from a specific promoter site. RNase H selectively degrades the RNA in an RNA-DNA hybrid [46]. A design of the SMART version of the reaction is shown in Fig. 4.

As seen in Fig. 4, during the first step of the reaction, Primer 1 binds to the Probe, and AMV-RT turns this hybrid into double stranded DNA. In this example, Primer 1 contains the T7 promoter sequence. The double stranded DNA serves as the template for T7 RNA polymerase, and the enzyme transcribes multiple strands of RNA. The RNA created has a different sequence than the original RNA in that region except for the segment in the center. Primer 2 then binds to the RNA strand and is extended by another AMV-RT step, creating a DNA-RNA hybrid. The RNA strand of this hybrid is then degraded by RNase H, yielding another Probe DNA strand and creating a cyclic exponential reaction.

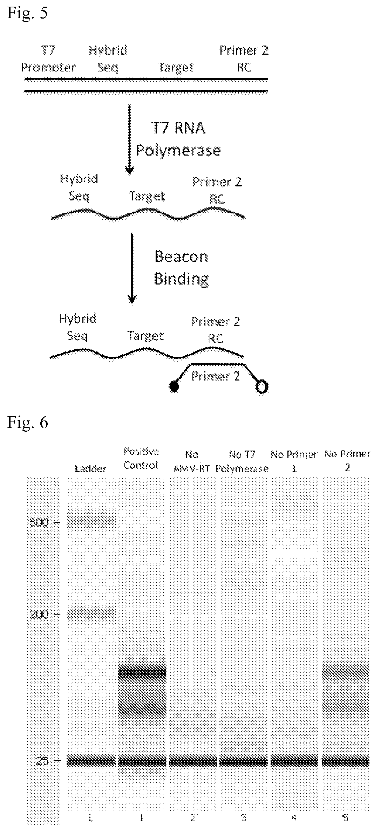

Real-time detection is achieved with molecular beacons or another sequence-specific fluorescent probe. As an example of a molecular beacon, one is created to bind at the Primer 2 binding site but with fewer nucleotides to bind to, thus Primer 2 will likely bind more readily to the site than the molecular beacon. In this example, noted is that the concentration of beacons is lower than Primer 2. Without being bound by any particular theory, it is believed that Fig. 5 shows how the beacon fluoresces in the assay. Furthermore, it will be understood by one skilled in the art that molecular beacons can be designed and bound at virtually any site.

A molecular beacon or another sequence-specific probe is useful to detect the amplification of RNA generated during the reaction. The beacon [or other detection method] can be specific to the sequence being detected by the assay (in which case multiple beacons may be used to differentiate between various sequences being assayed in a single reaction), or specific to the engineered sequences common to all the probes. A non-sequence specific method of assaying RNA synthesis (like RiboGreen) may also be used to evaluate the progression of the reaction.

The present method improves upon prior methods in fitting the expected shape of the RNA growth curve, an exponential reaction followed by a linear region by modelling the binding and enzymatic steps to create the expected curve shape.

### Materials and Methods

DNA SMART Probe, Primers, and Molecular Beacon

The SMART probe primers and molecular beacon were purchased from Integrated DNA Technologies, Inc. In some embodiments synthetic target DNA or RNA sequences instead of sequences isolated from clinical samples were employed. The probe portion complementary to the H5 viral RNA was chosen as it has been previously shown that it will bind to viral RNA [57]. Two iterations of the probe were created by varying the segment "Hybrid Seq RC." A second Primer 1 was made to hybridize to the new probe sequence. Primer 2 was shared for both sets of Primer 1 and SMART probe. Characterization of each of these strands and the resulting RNA was performed by using an online folding tool (UNAFold on The DINAMelt Server, RPI Bioinformatics). Analysis of binding between different strands was performed by the same software, assuming a 50 nM strand concentration, which is the concentration of molecular beacon used in the assay. The table below shows the self-binding data for each of the single stranded nucleic acid strands involved in the reaction (Table 1). Capitalized letters are variable and correspond to the ends of the probe, the primers, and the stem of the beacon. Lowercase letters are dependent on the target sequence of the probe,

Table 1: Self-binding thermodynamics for single stranded nucleic acids in the SMART assay.

| Identification | Sequence (5' to 3') | Length | dG (kcal/mol) | $T_m$ (°C) |
|---|---|---|---|---|
| Set 1 | | | | |
| Probe | TCAAGAGTAGACACAGGATCAGCATaggcaatagatggagtcacGTAATCAGATCAGAGCAATAGGTCA | 69 | -0.6 | 49.2 |
| Primer 1 | TAATACGACTCACTATAGGTGACCTATTGCTCTGATCTGATTAC | 44 | -1.4 | 56.4 |
| RNA made | GGUGACCUAUUGCUCUGAUCUGAUUACgugacuccaucuauugccuAUGCUGAUCCUGUGUCUACUCUUGA | 71 | -4.2 | 50.6 |
| Beacon 1 | /56-FAM/CGCGtcaagagtagacacaggatcCGCG/3IABlk_FQ/ | 28 | -1.4 | 54.5 |

(continued)

| Set 2 | | | | |
|---|---|---|---|---|
| Probe | TCAAGAGTAGACACAGGATCAGCATaggcaatagatggagt cacAGGCATATAGAGAGTCAGACAGGAG | 69 | -0.6 | 49.2 |
| Primer 1 | TAATACGACTCACTATAGGCTCCTGTCTGACTCTCT ATATGCCT | 44 | -1.0 | 50.5 |
| RNA made | GGCUCCUGUCUGACUCUCUAUAUGCCUgugacuccauc uauugccuAUGCUGAUCCUGUGUCUACUCUUGA | 71 | -5.2 | 50.3 |
| Beacon 2 | /56-FAM/CGTCGtcaagagtagacacaggatcaCGACG /3IABlk_FQ/ | 31 | -2.7 | 60.1 |
| Both Sets | | | | |
| Primer 2 | TCAAGAGTAGACACAGGATCAGCAT | 25 | 0.7 | 30.3 |

Table 2 characterizes the binding energies between the beacons and other single strands to ensure that the beacon would substantially bind to the RNA target. The data for both sets are shown with the far left column as the strand binding to the beacon. Note that the last line is the beacon binding to another strand of beacon and not itself, which is shown in Table 1.

Table 2: Binding energies between the molecular beacon and other single strand nucleic acids in the assay. The data for Set 1 are shown with Beacon 1, and the data for Set 2 are shown for Beacon 2.

| | Set 1 | | Set 2 | |
|---|---|---|---|---|
| Identification | dG (kcal/mol) | $T_m$ (°C) | dG (kcal/mol) | $T_m$ (°C) |
| Beacon-Probe | -4.3 | -1.8 | -3.3 | -8.6 |
| Beacon-Primer 1 | -3.7 | -17.9 | -5.3 | 14.9 |
| Bcacon-Primer 2 | -4.3 | -1.8 | -3.3 | -8.6 |
| Beacon-RNA made | -20.7 | 59.6 | -20.6 | 60.6 |
| Beacon-Beacon | -4.8 | 7 | -5.7 | 6.4 |

Each single stranded nucleic acid present in the assay was checked for binding to other single stranded molecules. Nearly all of the data show that there is minimal interaction between single stranded nucleic acids that are not engineered to bind to each other, except for a possible interaction between different strands of Primer 1.

Table 3 shows the data for each probe and primer set. Note that Set 2 was made specifically to address the possibility for Primer 1-Primer 1 interaction from the first step. Table 3: Binding energy between different Primer 1 strands in both Set 1 and Set 2.

| | Set 1 | | Set 2 | |
|---|---|---|---|---|
| Identification | dG (kcal/mol) | $T_m$ (°C) | dG (kcal/mol) | $T_m$ (°C) |
| Primer 1-Primer 1 | -8.5 | 36.7 | -4.2 | 16 |

Table 4 shows the binding energies between nucleic acids that should bind to each other. The data show that these strands should readily hybridize in the given conditions.

Table 4: Binding energies for the important binding steps for SMART amplification.

|  | Set 1 | | Set 2 | |
| --- | --- | --- | --- | --- |
| Identification | dG (kcal/mol) | $T_m$ (°C) | dG (kcal/mol) | $T_m$ (°C) |
| Probe-Primer 1 | -24 | 64.5 | -26.1 | 68.4 |
| Primer 2-RNA made | -26.3 | 68.3 | -26.3 | 68.3 |

*Performing the Assay in a Thermal Cycler and Gel Electrophoresis*

Three separate mixes are prepared: the reaction mix, the nucleic acid mix, and the enzyme mix. The concentrations for the reaction and enzyme mixes were based on Collies, et al. [44]. 10 μL of reaction mix is made with the following concentrations: 80 mM TrisHCl (pH 8.0), 24 mM $MgCl_2$, 140 mM KCl, 10 mM DTT, 2 mM each dNTP, 4 mM each rNTP, 30% DMSO, and 400 nM each primer. Salts were purchased from Ambion, DTT and NTPs were purchased from NEB, and DMSO was purchased from Sigma-Aldrich. 5 μL of nucleic acid mix contains variable concentrations of the ssDNA Probe in water. Heating and cooling is performed by a thermal cycler (MyCycler, Bio-Rad). The reaction mix and nucleic acid mix are added together and heated to 65°C for 5 min, and then the temperature is lowered to 41°C for 5 minutes to allow the temperature to reach the target temperature. 5 μL of enzyme mix is prepared with 1.3 U/μL AMV-RT, 0.5 U/μL RNase H, 6.4 U/μL T7 RNA polymerase, and 0.42 μg/μL bovine serum albumin (BSA) (Promega and NEB). The enzyme mix is added after the 5-minute equilibration period, and the reaction is carried out for 90 min at 41°C. The reaction is stopped by freezing the samples.

Gel electrophoresis is the first method used to analyze results. Two assays, the RNA Nano 6000 and Small RNA Assay, are both used on the Bioanalyzer 2100 (Agilent), a microfluidic chip platform for electrophoresis. The RNA 6000 assay is used as a qualitative assessment to determine whether amplification occurred, while the Small RNA assay is used to more precisely evaluate the size of RNA and DNA fragments generated during the reaction. Manufacturer's (Agilent) directions are followed as in the protocol for each assay, except that the samples used for the Small RNA Assay are diluted 1:5 because the assay is extremely sensitive to salt concentrations. The results are visualized in both a standard gel plot as well as electropherogram plots, generated from the program provided by Agilent for the Bioanalyzer 2100.

*Performing the Assay in a Fluorometer and Real- Time Detection*

The concentrations for the reaction mix, nucleic acid mix, and enzyme mix are the same as above except that molecular beacon is exchanged for some of the water in the reaction mix to give a 100 nM beacon concentration. The volumes of the mixes are increased to 40 μL of reaction mix, 20 μL of nucleic acid mix, and 20 μL of enzyme mix. The reaction mix and nucleic acid mix are first heated to 65°C for 5 minutes and then cooled to 41°C for 5 minutes in a thermal cycler. During this period, a new, disposable cuvette (UVette, Eppendorf) is placed into a fluorometer with a temperature control for each reaction. The temperature is set to 41°C, so that the cuvette itself can be equilibrated to the reaction temperature as much as possible. At the end of this period, the enzyme mix is added to the reaction. The cuvette is then removed from the holder, and the total reaction mix is transferred into the cuvette, which is inspected to ensure that no bubbles appear. The cuvette is placed back into the fluorometer, and the reaction is run for 90 minutes.

The fluorometer is set to an absorbance of 494 nm and an emission of 518 nm. This data is the peak absorbance and emission for the fluorescent molecule, 6-FAM [58]. The fluorometer is set to acquire data every 5 minutes, starting at time 0 minutes. Five data points are taken for each time point. The data from the first time point, 0 minutes, is not presented in the final results as it was found that there is a spike in fluorescence between the first two time points, probably due to the mixes and cuvette equilibrating to 41°C. The reaction is run for 90 minutes. The data are plotted using a MATLAB script to average the 5 data points for each time point, remove the 0-minute time point, scale all time points to the 5-minute time point, and subtracting 1 so that the first time point is 0. Data are plotted as relative fluorescence against time.

*Qatantification and Modeling of SMART amplification and real-time detection*

A model for the amplification step and real-time detection using molecular beacons was created using MATLAB (The Mathworks, Inc.) and Mathematica (Wolfram Research, Inc.) in order to iterate through the steps of the reaction to perform the calculations. The data is presented with the following notations:

Pr is Probe,

P1 is Primer 1,
P2 is Primer 2,
B is beacon,
RNA is the RNA made, and
dsDNA is the double-stranded DNA that serves as the template for T7 RNA polymerase.

A complex of two of these species is denoted by placing the shorthand names together (i.e. PrP1 stands for Probe and Primer 1 bound together). The reaction was separated into 6 different steps:

1. $\text{Pr} + \text{P1} \rightarrow \text{PrP1}$

2. $\text{PrP1} \xrightarrow{\;AMV-RT\;} \text{dsDNA}$

3. $\text{dsDNA} \xrightarrow{\;T7\;} \text{dsDNA} + \text{RNA}$

4a. $\text{P2} + \text{RNA} \rightarrow \text{P2RNA}$

4b. $\text{B} + \text{RNA} \rightarrow \text{BRNA}$

5. $\text{P2RNA} \xrightarrow{\;AMV-RT\;} \text{DNARNA}$

6. $\text{DNARNA} \xrightarrow{\;RNaseH\;} \text{Pr}$

All other events are considered to be negligible. In general, there were two types of steps: binding steps and enzymatic steps. The binding steps (1 and 4) can be described in similar ways. The equation for step 1 is shown below, where x is the change in concentration of Pr or P1 during a small time step, and $k_1$ is the equilibrium constant for step 1 (Eq. 1):

$$\frac{[\text{Pr}P1]+x}{([\text{Pr}]-x)([P1]-x)} = k_1 \tag{1}$$

After finding x, the concentrations of the species are adjusted by the concentration x. Step 4 involves competitive binding of Primer 2 and Beacon to the RNA molecules. It differs from step 1 as two equations must be solved simultaneously. Equations 2 and 3 describe these binding events, where $x$ is the decrease in concentration of Primer 2 or the increase of the complex Primer 2-RNA, and $y$ is the decrease in concentration of Beacon or the increase of the complex Beacon-RNA, and the sum of $x$ and $y$ represents the decrease in concentration of RNA.

$$\frac{[P2RNA]+x}{([P2]-x)([RNA]-x-y)} = k_{4a} \tag{2}$$

$$\frac{[BRNA]+x}{([B]-x)([RNA]-x-y)} = k_{4b} \tag{3}$$

The enzymatic steps are all described in similar ways as well, using the Michaelis-Menten equation. One assumption is that the two enzymes that polymerize nucleic acids, AMV-RT and T7 RNA polymerase, are saturated with NTPs through the reaction. Calculations show that this is true during the period of beacon binding. For rNTPs, a base count on the RNA created in the reaction shows that U is the most used base. Since no RNA is in the sample in the beginning, the following is noted:

$$\frac{1 RNA}{27 UTP} \cdot 2mM\, UTP = 0.0741 mM \cdot RNA = 74.1 \mu M \cdot RNA \tag{4}$$

Thus, 74.1 $\mu$M of RNA is made. This is on the order of $10^3$ greater than the beacon concentration (50 nM), so rNTPs are not limiting. Again, without being A similar analysis can be done for dNTPs. For a conservative estimate, assume that the entire Probe begins as Primer 2, and thus the double stranded DNA starts from Primer 2 and Primer 1. A base count shows that T is the limiting base for the reaction. Thus, a similar equation can be used as above:

$$1mM \cdot TTP \frac{1dsDNA}{31TTP} = 0.0323mM \cdot dsDNA = 32.3\mu M \cdot dsDNA \qquad (5)$$

By this calculation a 32.3 μM of DNA is created. Since multiple strands of RNA can be made from one template of DNA, it is probable that rNTPs will be used in polymerization reactions before dNTPs, so dNTPs are not believed limiting in comparison to the beacon.

Since the enzymes are saturated with NTPs, the Michaelis-Menten equation collapses into the following form [59]:

$$V = \frac{V_{max}[S]}{K_M + [S]} = \frac{k_{cat}[E][S]}{K_M + [S]} \qquad (6)$$

where $V_{max}$ and $K_M$ are the Michaelis-constants, $k_{cat}$ is the general rate constant for a reaction or the limiting step of it, [E] is the enzyme concentration, and [S] is the substrate concentration. Given the assumption above, the substrate is treated as the nucleic acid to which the enzyme binds rather than the NTPs. For each iteration, V is multiplied by the iteration time step so that the actual change in concentration of the substrate can be calculated for an iteration. From this, the concentration of the substrate and product involved in the enzymatic reaction is changed by the amount calculated for the iteration. Equation 6 is the general equation used for all of the enzyme steps (2, 3, 5 and 6).

The main assumption for the Michaelis-Menten equation is the steady-state assumption, which states that [ES], or the concentration of the enzyme bound to the substrate, does not change with time [60]. In the reaction as a whole, this is not true. Since the reaction is carried out iteratively, this statement can be treated as approximately true for each iteration, and thus the velocity of the reaction will change with time.

To solve the equation, $k_{cat}$, $K_M$, and [E] are needed. For each enzyme, values were found in literature for $k_{cat}$ and $K_M$, and in some cases $k_{cat}$ is found by using the relation that $k_{cat} = V_{max}[E]$. Since this reaction is different from other reaction conditions in literature, the final values used were estimated. For AMV-RT, it has been shown that the initiation step is the rate limiting step [61]. Therefore, the values for initiation were used. These values vary depending on the identity of the end base pair. For this reason an average was taken, with the value of $k_{cat}$ as $5.625*10^5$ s$^{-1}$ and $K_M$ as $8.5*10^{-8}$ M [62]. To estimate $k_{cat}$ for T7 RNA polymerase, Arnold *et al.* provided values for $k_{eff}$, which describes the amount of nucleotides transcribed per time while taking initiation, elongation, and termination into account. According to Arnold, the rate constant can vary between about 5 s$^{-1}$ and 630 s$^{-1}$ for the plasmids they used. The RNA created in the assay is much shorter than a plasmid. As such it is believed that initiation and termination play a larger role in those examples. Considering that these are more likely to be the limiting factor, a small rate constant is chosen, 5 s$^{-1}$, which is divided by 71, the length of the RNA made, to retrieve the final $k_{cat}$, which describe the number of molecules made per second. Thus, the value chosen for $k_{cat}$ was $7.04*10^{-2}$ s$^{-1}$, and $K_M$ was used as in the paper, $6.3*10^{-9}$ M [59]. For RNase H, many values were provided depending on the bases present. As with, AMV-RT, the average of these values were taken. The value for $k_{cat}$ used was $1.6*10^{-1}$ s$^{-1}$ and the value for $K_M$ was $7.32*10^{-8}$ M. The values for $k_{cat}$ and $K_M$ used in the model are summarized below (Table 5).

Table 1: Kinetic data used in the model for each enzyme in the SMART amplification reaction.

| Enzyme | $k_{cat}$ (s$^{-1}$) | $K_M$ (M) |
|---|---|---|
| AMV-RT | $1.9369 * 10^{-2}$ | $8.5 * 10^{-8}$ |
| T7 RNA Polymerase | $7.04 * 10^{-2}$ | $6.3 * 10^{-9}$ |
| RNase H | $1.6 * 10^{-1}$ | $7.32 * 10^{-8}$ |

Values for [E] were calculated using the known number of Units/volume of each enzyme in the reaction mix according to the protocol along with specific activity and molecular weight (values were obtained from New England Biolabs) These values permitted conversion of Units/volume into concentrations.

Table 2: Data describing important parameters for each enzyme for the SMART amplification reaction.

| Enzyme | Units/volume (U/L) | Specific Activity (U/mg) | Molecular Weight (Da) (g/mol) | [E] (M) |
|---|---|---|---|---|
| AMV-RT | $3.25 * 10^5$ | $4 * 10^4$ | $1.58 * 10^5$ | $5.14 * 10^{-8}$ |
| T7 RNA Polymerase | $1.6 * 10^6$ | $7.4 * 10^5$ | $9.8 * 10^4$ | $2.21 * 10^{-7}$ |

(continued)

| Enzyme | Units/volume (U/L) | Specific Activity (U/mg) | Molecular Weight (Da) (g/mol) | [E] (M) |
|---|---|---|---|---|
| RNase H | $5 * 10^3$ | $1.1 * 10^6$ | $1.85 * 10^4$ | $2.46 * 10^{-10}$ |

The final parameter to be chosen is the time step. Since the binding steps do not contain the value $t$ for the length of an iteration of a time step, the time step represents approximately how long it takes for the binding events to reach equilibrium. For solution-phase kinetics, DNA with minimal secondary structure will nearly completely equilibrate by 60 seconds [63]. Thus, the time step chosen for the model is 60 seconds.

Results

The initial test checked if the amplification reaction was correctly designed and could be a viable option. In this experiment, Set 1 was used. A positive control was a reaction that contained all of the reagents along with 100 nM of SMART Probe in the final concentration. The other conditions involved removing one of the following: AMV-RT, T7 RNA polymerase, Primer 1, and Primer 2. Gel electrophoresis using the RNA Nano 6000 assay was run, and the gel plot is shown Fig. **6.**

The first lane in Fig. 6 shows the positive control. Two dark bands show that a high concentration of nucleic acid was present. Two peaks are shown. The RNA Nano 6000 assay did not separate small strands of nucleic acids, less than 100 bases. Removing either of the enzymes or Primer 1 shows that the amplification does not occur when any of these reagents are missing. This agrees with the design of the assay, as AMV-RT was needed to make the template for RNA transcription. T7 RNA polymerase was the enzyme involved in transcription, and Primer 1 contained the promoter sequence for the T7 polymerase. Thus, absent any of these reagents, RNA was not polymerized. The final lane, which does not contain Primer 2 in the reaction, shows that while amplification occurred, it did so at a diminished rate. This is shown as the bands were lighter, and fewer nucleic acids were made compared to the positive control. This also follows the design as removing Primer 2 did not allow for a cyclic, exponential reaction, but it still allowed for RNA transcription. It is important to note that 100 nM Probe was a relatively high concentration, and thus Primer 2 is still an important part of the reaction for lower concentrations of Probe.

To optimize some of the conditions of the reaction, DMSO concentration and TrisHCl buffer pH were varied. For both experiments, Set 1 was used. DMSO is believed to reduce nonspecific interaction between different nucleic acids as well as disrupting secondary structure of the template and primers [52]. DMSO is also reported as difficult to work with as reagents can precipitate out of solution. Since the length of the product has been decreased, it was hypothesized that a lower DMSO concentration could be a viable option. The reaction was performed with the normal 15% DMSO final concentration and with 5% DMSO final concentration in a thermal cycler and was analyzed with gel electrophoresis with the RNA Nano 6000 assay

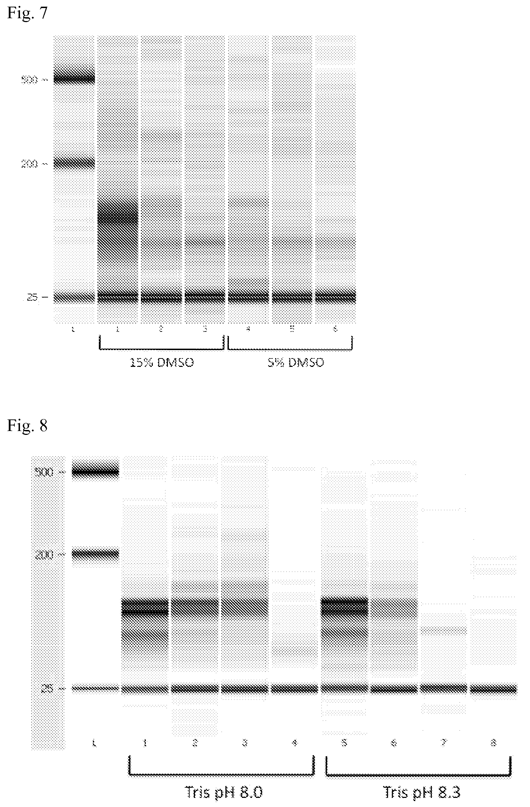

As shown in Fig. 7, three concentrations of probe, 100 nM, 10 nM, and 1 nM, were used for the different DMSO concentrations. The gel plot shows that the reaction was hindered by 5% DMSO. Throughout all of the experiments, even if some reagents precipitated out of solution at 15% DMSO, heating and mixing could put them back into solution and did not appear to affect the results of the reaction.

Initial tests used Tris-HCl pH 8.0, but others have reported using Tris-HCl pH 8.3 and 8.5 [44, 54]. As an experiment, Tris-HCl pH was also varied at pH 8.0 and pH 8.3. Three concentrations of probe, 100 nM, 10 nM, and 1 nM, and a negative control, 0 nM probe, were run at each pH level. The results of the gel plot are shown in Fig. 7. Lanes 1-4 correspond to Tris-HCl pH 8.0, and lanes 5-8 with pH 8.3.

The results as shown in Fig. 8 are that although the reaction continues to work at the higher pH level, the efficiency of the reaction drops due to the higher pH. Because of this, Tris-HCl pH 8.0 was used for all subsequent reactions to maintain efficiency.

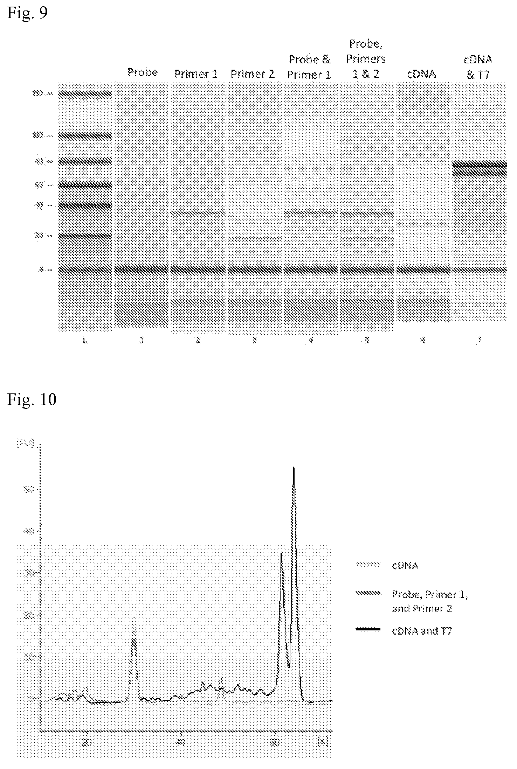

Further studies were carried out to identify each individual strand as shown in Fig. 8 on a gel or electropherogram plot. For this purpose, the Small RNA Assay from Agilent was used in various experiments. Set 1 was used for this experiment. To identify known products, the individual DNA strands in the reaction, Probe, Primer 1, Primer 2, and combinations of the primers were added to a reaction mix without enzymes. In addition, the double stranded DNA expected from the reaction after the AMV-RT step was purchased and added into a mix without enzymes and with only the T7 RNA polymerase to identify the double stranded template DNA as well as the RNA produced in the reaction. Probe concentration was 10 nM in the reaction. The gel plot, Fig. 9, shows that the starting concentration of Probe is too low to be detected via gel electrophoresis. In addition, the combination of Probe and Primer 1 bound together show up where Primer 1 alone appears. Adding Primer 2 to this mix simply adds a band where expected for Primer 2, which seems to confirm that Primer 2 is not binding in significant amounts to either the Probe or Primer 1. Given the known lengths of these segments, the bands seem to come more quickly than the assay calculates via the ladder in the left most lane. This could be due to the high salt content of the reaction conditions. Furthermore, the cDNA band comes even quicker than expected. Thus, double-stranded species come quicker than expected, most likely due to their minimal secondary structure, which would slow their migration through the gel. The final lane shows that multiple RNA products are actually made, as there are two strong bands that appear when T7 is simply added to cDNA. Abortive products are not uncommon with RNA polymerases, and thus this is not a cause for concern. The electropherogram of this data, Fig. 10, shows a clearer diagram of the last three lanes. In the data, the signal for the cDNA and T7 lane becomes jagged, rising a little over the baseline. This implies that other, smaller RNA or DNA products were also made during the reaction, likely as a result of incomplete transcription from a template.

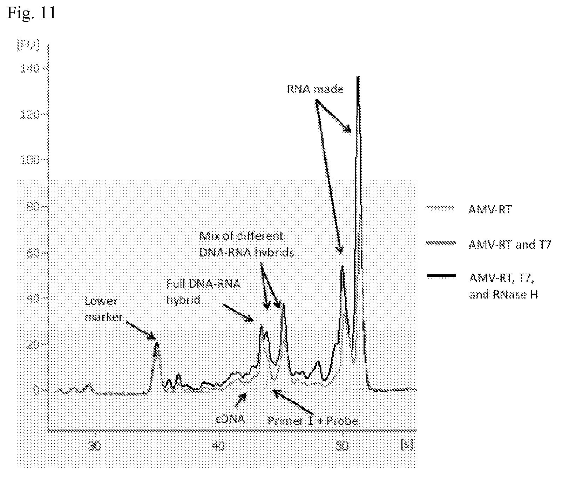

The above data are shown in the electropherogram of Fig. 11. Without being bound by any particular theory it is believed that Fig. 11 establishes an increase in efficiency when RNase H is added despite a relatively high starting concentration of Probe and that RNase H facilitates the reaction. Known strands are marked on Fig 11. Fig. 11 confirms that the reaction makes the RNA strand that is designed to be made.

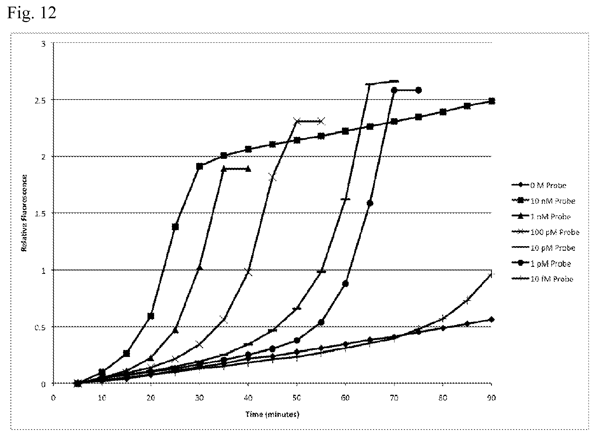

Fluorometer data was taken by using a series of 10:1 dilutions of SMART probe. Both sets were tested. Negative control was taken as 0 M SMART probe. The data for Set 1 is shown in Fig. 12. For the curves corresponding to starting concentrations of Probe of 1 nM, 100 pM, 10 pM, and 1 pM, the lines flatten at the top. This occurred because the detector of the fluorometer became saturated. Since relative fluorescence is plotted, though, and the background signal at 5 minutes varied, the curves peak at different points. Fig. 12 shows that decreases in starting concentration of Probe lead to increased time to achieve the same level of fluorescence. In the instant assay, the lower threshold for detection is about 100 fM Probe. Fig 12 also shows that a criterion for a positive signal is safely set at about three times the original background signal (corresponding with a relative fluorescence of 2). Flourometric data is useful in both quantitative and quantitive measurement,

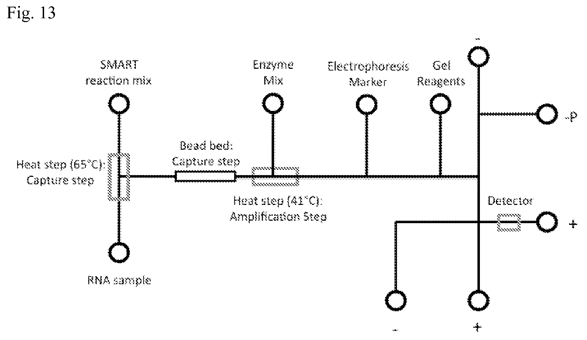

A microfluidic chip incorporating the present method allows for a cost-effective option as minimal reagents are used and facilitating uses in a point of care device. A chip design is presented in Fig 13. This example incorporates a section for the capture step in addition to the amplification step.

In Fig 13, gray areas in the schematic denote apparatuses for either heating at a constant temperature (two on the left) or for a detector (right). In this schematic, the left side will vary pressures so that plugs of samples will sit in the heating and capture portions of the chip for appropriate lengths of time.

The right side is shown with -P for negative pressure and + and - for positive and negative charges for the electrophoresis portion of the assay.

The instant assay for RNA detection provides an alternative to NASBA, particularly where NASBA is ineffective due to molecular constraints. The assay also provides advantages over PCR that allows this technique to be translated to a point of care device. The novel addition to NASBA of amplifying a variable DNA probe gives this technique a solution to secondary structure issues that can occur in amplification processes.

1. Chan, P.K.S., Outbreak of Avian Influenza A(H5N1) Virus Infection in Hong Kong in 1997. Clinical Infectious Diseases, 2002. 34: p. S58-S60.

2. Bridges, C.B., et al., Prevention and control of influenza: recommendations of the Advisory Committee on Immunization Practices (ACIP). Morbidity & Mortality Weekly Report, 2002. 51(RR-3): p. 1-31.

3. Thompson, W.W., et al., Mortality associated with influenza and respiratory syncytial virus in the United States. Journal of the American Medical Association, 2003. 289: p. 179-86.

4. Webster, R.G., et al., Evolution and Ecology of Influenza A Viruses. Microbiological Reviews, 1992. 56(1): p. 152-179.

5. Hinshaw, V.S., R.G. Webster, and B. Turner, The perpetuation of orthomyxoviruses and paramyxoviruses in Canadian waterfowl. Canadian Journal of Microbiology, 1980. 26: p. 622-629.

6. Cox, N.J., Z.S. Bai, and A.P. Kendal, Laboratory-based surveillance of influenza A (H1N1) and A (H3N2) viruses in 1980-81: antigenic and genomic analysis. Bulletin of the World Health Organization, 1983. 61(1): p. 143-152.

7. Webster, R.G., C.H. Campbell, and A. Granoff, The "in vivo" production of "new" influenza A viruses. Virology, 1971. 44: p. 317-328.

8. Shortridge, K.F., The next pandemic influenza virus? The Lancet, 1995. 346: p. 1210-12.

9. Tang, X., et al., 1998. Chinese Journal of Animal and Poultry Infectious Diseases, Isolation and characterization of prevalent strains of avian influenza viruses in China. 20: p. 1-5.

10. Ligon, B.L., Avian Influenza Virus H5N1: A Review of Its History and Information Regarding Its Potential to Cause the Next Pandemic. Seminars in Pediatric Infectious Diseases, 2005. 16: p. 326-335.

11. WHO, Avian Influenza: assessing the pandemic threat. WHO/CDS, 2005.

12. Yuen, K.Y., et al., Clinical features and rapid viral diagnosis of human disease associated with avian influenza A H5N1 virus. The Lancet, 1998. 351: p. 467-471.

13. WHO, Avian Influenza A (H5N1) Infections in Humans. The New England Journal of Medicine, 2005. 353(13): p. 1374-85.

14. Couch, R.B., Prevention and Treatment of Influenza. The New England Journal of Medicine, 2000. 343: p. 1778-1787.

15. La Montagne, J.R., et al., Summary of clinical trials of inactivated influenza vaccine. Reviews of Infectious Diseases, 1983. 5: p. 723-736.

16. Parkman, P.D., et al., Summary of clinical trials of influenza virus vaccines in adults. Journal of Infectious Diseases, 1977. 136:Suppl: p. S722-S730.

17. Gross, P.A., et al., The Efficacy of Influenza Vaccine in Elderly Persons: A Meta-Analysis and Review of the Literature. Annals of Internal Medicine, 1995. 123(7): p. 518-527.

18. Kroon, F.P., et al., Antibody response to influenza, tetanus and pneumococcal vaccines in HIV-seropositive individuals in relation to the number of CD4+ lymphocytes. AIDS, 1994. 8(469-476).

19. Demicheli, V., et al., Prevention and early treatment of influenza in healthy adults. Vaccine, 2000. 18(957-1030).

20. WHO, Evolution of avian influenza viruses in Asia. Emerging Infectious Diseases, 2005. 11: p. 1515-21.

21. Stephenson, I., et al., Development of vaccines against influenza h5. The Lancet Infectious Diseases, 2006. 6(8): p. 458-460.

22. Stephenson, I., and K.G. Nicholson, Influenza: vaccination and treatment. European Respiratory Journal, 2001. 17: p. 1282-1293.

23. Hayden, F.G., Antivirals for influenza: Historical perspectives and lessons learned. Antiviral Research, 2006. 71: p. 372-378.

24. Sabin, A.B., and G.G. Jackson, Amantadine and Influenza: Evaluation of Conflicting Reports. The Journal of Infectious Diseases, 1978. 138(4): p. 557-568.

25. Bright, R.A., et al., Adamantane Resistance Among Influenza A Viruses Isolated Early Duping the 2005-2006 Influenza Season in the United States. The Journal of the American Medical Association, 2006. 295(8): p. 891-894.

26. Hurt, A.C., et al., Susceptibility of highly pathogenic A (H5N1) avian influenza viruses to the neuraminidase inhibitors and adamantanes. Antiviral Research, 2007. 73: p. 228-231.

27. Gubareva, L.V., Molecular mechanisms of influenza virus resistance to neuraminidase inhibitors. Virus Research, 2004. 103: p. 199-203.

28. Woo, P.C.K., et al., Cost-Effectiveness of Rapid Diagnosis of Viral Respiratory Tract Infections in Pediatric Patients. Journal of Clinical Microbiology, 1996. 35(6): p. 1579-1581.

29. Bonner, A.B., et al., Impact of the Rapid Diagnosis of Influenza on Physician Decision-Making and Patient Management in the Pediatric Emergence Department: Results of a Randomized, Prospective, Controlled Trial. Pediatrics, 2003. 112: p. 363-367.

30. Falsey, A.R., Y. Murata, and E. E. Walsh, Impact of Rapid Diagnosis on Management of Adults Hospitalized With Influenza. Archives of Internal Medicine, 2007. 167: p. 354-360.

31. Zambon, M., et al., Diagnosis of Influenza in the Community. Archives of Internal Medicine, 2001. 161: p. 2116-2122.

32. CDC. Role of laboratory diagnosis. 8 March 2010; Available from: <http://www.cdc.gov/flu/professionals/diagnosis/labrole.htm>.

33. Petric, M., L. Comanor, and C. A. Petti, Role of the Laboratory in Diagnosis of Influenza during Seasonal Epidemic and Potential Pandemic. The Journal of Infectious Diseases, 2006. 194: p. S98-110.

34. Fedorko, D.P., et al., Performance of Rapid Tests for Detection of Avian influenza A Virus Types H5N1 and H9N2. Journal of Clinical Microbiology, 2006. 44(4): p. 1596-1597.

35. Gavin, P.J., and R. B. Thomson, Jr., Review of Rapid Diagnostic Tests for Influenza. Clinical and Applied Immunology Reviews, 2003. 4: p. 151-172.

36. Demmler, G.J., Laboratory Diagnosis of Influenza: Recent Advances. Seminars in Pediatric Infectious Diseases, 2002. 13(2): p. 85-90.

37. Dunn, J.J., et al., Comparison of the Denka-Seiken INFLU A.B.-Quick and BD Directigen Flu A+B kits with direct fluorescent antibody staining and shell vial culture methods for rapid detection of influenza viruses. Journal of Clinical Microbiology, 2003. 41(5): p. 2180-2183.

38. Saiki, R.K., et al., Enzymatic amplification of beta-globin genomic sequences and restriction site analysis for diagnosis of sickle cell anemia. Science, 1985. 230(4732): p. 1350-1354.

39. Schochetman G., C. Ou, and W. K. Jones, Polyermase Chain Reaction. The Journal of Infectious Diseases, 1988. 158(6): p. 1154-1157.

40. Saiki, R.K., et al., Primer-directed enzymatic amplificaiton of DNA with a thermostable DNA polymerase. Science, 1988. 239(4839): p. 487-491.

41. Ellis, J.S., and M. C. Zambon, Molecular diagnosis of influenza. Reviews in Medical Viorlogy, 2002. 12: p. 375-389.

42. Payungporn, S., et al., Single step multiplex real-time RT-PCR for H5N1 influenza A virus detection. Journal of Virological Methods, 2006. 131: p. 143-147.

43. Poddar, S.K., Influenza virus types and subtypes detection by single step single tube multiplex reverse transcription-polyermase chain reaction (RT-PCR) and agarose gel electrophoresis. Journal of Virological Methods, 2002. 99(63-70).

44. Collins, R.A., et al., Detection of highly pathogenic and low pathogenic avian influenza subtype H5 (Eurasian lineage) using NASBA. Journal of Virological Methods, 2002. 103: p. 213-225.

45. Guatelli, J.C., et al., Isothermal, in vitro amplification of nucleic acids by a multienzyme reaction modeled after retroviral replication. Proceedings of the National Academy of Sciences, 1990. 87: p. 1874-1878.

46. Compton, J., Nucleic acid sequence-based amplification. Nature, 1991. 350: p. 91-92.

47. Gulliksen, A., et al., Parallel nanoliter detection of cancer markers using polymer microchips. The Royal Society of Chemistry, 2005. 5: p. 416-420.

48. Zaaijer, H.L., et al., Detection of HIV-1 RNA in plasma by isothermal amplification (NASBA) irrespective of the stage of HIV-1 infection. Journal of Virological Methods, 1995. 52: p. 175-181.

49. Lau, L., et al., Nucleic acid sequence-based amplification methods to detect avian influenza virus. Biochemical and Biophysical Research Communications, 2004. 313: p. 336-342.

50. Jean, J., D.H. D'Souza, and L. Jaykus, Multiplex Nucleic Acid Sequence-Based Amplification for Simultaneous Detection of Several Enteric Viruses in Model Ready-To-Eat Foods. Applied and Environmental Microbiology, 2004. 70(11): p. 6603-6610.

51. Jean, J., et al., Simultaneous detection and identification of hepatitis A virus and rotavirus by multiplex nucleic acid sequence-based amplification (NASBA) and microtiter plate hybridization system. Journal of Virological Methods, 2002. 105(1): p. 123-132.

52. Deiman, B., P. van Aarle, and P. Sillekens, Characteristics and Applications of Nucleic Acid Sequence-Based Amplification (NASBA). Molecular Biotechnology, 2002. 20: p. 163-179.

53. Miao, W., Electrogenerated Chemiluminescence and Its Biorelated Applications. Chemical Reviews, 2008. 108(7): p. 2506-2553.

54. Leone, G., et al., Molecular beacon probes combined with amplification by NASBA enable homogeneous, real-time detection of RNA. Nucleic Acids Research, 1998. 26(9): p. 2150-2155.

55. Tyagi, S., and F.R. Kramer, Molecular Beacons: Probes that Fluoresce upon Hybridization. Nature Biotechnology, 1996. 14: p. 303-308.

56. Weusten, J.J.A.M., et al., Principles of quantitation of viral loads using nucleic acid sequence-based amplification in combination with homogeneous detection using molecular beacons. Nucleic Acids Research, 2002. 30(6): p. 1-7.

57. Kerby, M., et al., Direct Sequence Detection of Structured H5 Influenza Viral RNA. The Journal of Molecular Diagnostics, 2008. 10(3): p. 225-235.

58. Behlke, M.A., et al. Fluorescence and Fluorescence Applications. 2005; Available from: https://3979.voxcdn.com/Support/Technical/TechnicalBulletinPDF/Fluorescence_and_F1 uorescence_Applications.pdf.

59. Arnold, S., et al., Kinetic Modeling and Simulation of In Vitro Transcription by Phage T7 RNA Polymerase. Biotechnology and Bioengineering, 2000. 72(5): p. 548-561.

60. Nelson, D.L., and M. M. Cox, Lehninger: Principles of Biochemistry. 2008, New York: W.H. Freeman and Company.

61. Berger, S.L., et al., Reverse Transcriptase and Its Associated Ribonuclease H: Inteiplay of Two Enzyme Activities Controls the Yield of Single-Stranded Complementary Deoxyribonucleic Acid. Biochemistry, 1983. 22: p. 2365-2372.

62. Mendelman, L.V., J. Petruska, and M. F. Goodman, Base Mispair Extension Kinetics. The Journal of Biological Chemistry, 1990. 265(4): p. 2338-2346.

63. Gao, Y., L. K. Wolf, and R. M. Georgiadis, Secondary structure effects on DNA hybridization kinetics: a solution versus surface comparison. Nucleic Acids Research, 2006. 34(11): p. 3370-3377.

## Claims

1. A method of amplifying Target nucleotide sequences comprising the steps of

   (i) introducing to target nucleotide sequences two or more probe nucleotide sequences under hybridizing conditions;
   wherein each said probe nucleotide sequence comprises at least three regions being,
   a Primer 1 comprising a Hybrid Seq RC region; and,
   a specific second region, being a Target RC region contiguous with said Primer 1 region; and
   a third region being a Primer 2 region contiguous with said second region; and,
   (ii) selectively amplifying said Target nucleotide sequences;
   wherein Primer 1 means a primer comprising a 5' promoter region for an RNA polymerase that catalyzes the formation of RNA in the 5' → 3' direction; Hybrid Seq RC means a sequence on the 3' end of the Probe sequence that is sufficiently complementary to the 3' end of Primer 1 to hybridize under reaction conditions to enable the creation of a double-stranded DNA sequence by a DNA-dependent DNA polymerase; Target RC means a short oligonucleotide sequence that is reverse complementary to a sequence on a Target nucleotide sequence; and Primer 2 means an oligonucleotide sequence complementary to the 3' end of RNA transcribed from the DNA template synthesized in Primer 1, which will hybridize to this sequence to permit the transcription of a DNA:RNA hybrid catalyzed by an RNA-dependent DNA polymerase.

2. The method of Claim 1 further comprising detecting said amplified nucleotide sequences.

3. The method of Claim 2 wherein said detecting is by the method of gel electrophoresis or fluorescence.

4. The method of Claim 3 wherein said detection by fluorescence is by molecular beacon.

5. The method of any one of Claims 1 to 4 wherein said amplifying of step (ii) comprises transcribing hybridized Target RNA into double-stranded DNA .

6. The method of Claim 5 wherein a promoter containing a T7 promoter sequence binds to said probe RNA.

7. The method of Claim 5 or 6 wherein said transcribing is by means of a reverse transcriptase, preferably by AMV-RT.

8. The method of any one of Claims 5 to 7 further comprising transcribing said resulting double stranded DNA into RNA.

9. The method of Claim 8 wherein said transcribing is by means of an RNA polymerase that catalyzes the formation of RNA in the 5'→ 3' direction polymerase forming a two strand, DNA-RNA hybrid.

10. The method of Claim 9 wherein said polymerase is a T7 RNA polymerase.

11. The method of Claim 9 or 10 wherein only said RNA strand of a DNA-RNA hybrid is degraded.

12. The method of Claim 11 wherein said degradation of said RNA strand is by RNase H.

13. The method of any one of Claims 1 to 12 wherein said Hybrid Seq RC region, Target RC region and said Primer 2 region each comprise from about 8 to about 35 bases which number as to each region may be the same or different.

14. The method of any one of Claims 1 to 13 further comprising binding a molecular beacon to the amplified RNA.

## Patentansprüche

1. Verfahren zum Amplifizieren von Ziel-Nukleotidsequenzen, umfassend die Schritte des

(i) Einbringens in Ziel-Nukleotidsequenzen zweier oder mehrerer Sonden-Nukleotidsequenzen unter hybridisierenden Bedingungen;
wobei jede besagte Sonden-Nukleotidsequenz mindestens drei Regionen umfasst, die sind:

ein Primer 1, umfassend eine HybridSeq-RC-Region; und
eine spezifische zweite Region, die eine Ziel-RC-Region, zusammenhängend mit der Primer 1-Region, ist; und
eine dritte Region, die eine Primer 2-Region, zusammenhängend mit der zweiten Region, ist; und

(ii) selektiven Amplifizierens dieser Ziel-Nukleotidsequenzen;
wobei Primer 1 einen Primer meint, umfassend eine 5'-Promotorregion für eine RNA-Polymerase, welche die Bildung von RNA in der 5' → 3'-Richtung katalysiert; HybridSeq-RC eine Sequenz am 3'-Ende der Sondensequenz meint, die ausreichend komplementär zu dem 3'-Ende von Primer 1 ist, um unter Reaktionsbedingungen zu hybridisieren, um die Schaffung einer doppelsträngigen DNA-Sequenz durch eine DNA-abhängige DNA-Polymerase zu ermöglichen; Ziel-RC eine kurze Oligonukleotidsequenz meint, die umgekehrt (revers) komplementär zu einer Sequenz an einer Ziel-Nukleotidsequenz ist; und Primer 2 eine Oligonukleotidsequenz meint, die zu dem 3'-Ende der RNA, die von der in Primer 1 synthetisierten DNA-Template transkribiert wird, komplementär ist, die an diese Sequenz hybridisieren wird, um die Transkription eines DNA:RNA-Hybrids, katalysiert durch eine RNA-abhängige DNA-Polymerase, zu ermöglichen.

2. Verfahren gemäß Anspruch 1, außerdem umfassend das Nachweisen der amplifizierten Nukleotidsequenzen.

3. Verfahren gemäß Anspruch 2, wobei das Nachweisen mittels des Verfahrens der Gelelektrophorese oder Fluoreszenz erfolgt.

4. Verfahren gemäß Anspruch 3, wobei der Nachweis durch Fluoreszenz mittels eines Molecular Beacon erfolgt.

5. Verfahren gemäß jedem der Ansprüche 1 bis 4, wobei das Amplifizieren des Schritts (ii) das Transkribieren der hybridisierten Ziel-RNA in doppelsträngige DNA umfasst.

6. Verfahren gemäß Anspruch 5, wobei ein Promotor, der die T7-Promotorsequenz enthält, an die Sonden-RNA bindet.

7. Verfahren gemäß Anspruch 5 oder 6, wobei das Transkribieren mittels einer Reversen Transkriptase, vorzugweise mittels AMV-RT, erfolgt.

8. Verfahren gemäß jedem der Ansprüche 5 bis 7, außerdem umfassend das Transkribieren der resultierenden doppelsträngigen DNA in RNA.

9. Verfahren gemäß Anspruch 8, wobei das Transkribieren mittels einer RNA-Polymerase erfolgt, welche die Bildung der RNA in der 5'→ 3'-Richtung katalysiert, wobei die Polymerase ein doppelsträngiges DNA:RNA-Hybrid bildet.

10. Verfahren gemäß Anspruch 9, wobei die Polymerase eine T7 RNA-Polymerase ist.

11. Verfahren gemäß Anspruch 9 oder 10, wobei lediglich der RNA-Strang eines DNA:RNA-Hybrids abgebaut wird.

12. Verfahren gemäß Anspruch 11, wobei der Abbau des RNA-Strangs durch RNase H erfolgt.

13. Verfahren gemäß jedem der Ansprüche 1 bis 12, wobei die HybridSeq-RC-Region, Ziel-RC-Region und die Primer 2-Region jeweils von etwa 8 bis etwa 35 Basen umfasst, welche Anzahl bezüglich jeder Region gleich oder verschieden sein kann.

14. Verfahren gemäß jedem der Ansprüche 1 bis 13, außerdem umfassend das Binden eines Molecular Beacon an die amplifizierte RNA.

**Revendications**

1. Procédé d'amplification de séquences nucléotidiques cibles, comprenant les étapes suivantes

(i) l'introduction dans les séquences nucléotidiques cibles de deux ou plus de deux séquences nucléotidiques de sonde dans des conditions d'hybridation ;
dans laquelle chaque séquence nucléotidique de sonde susmentionnée comprend au moins trois régions qui sont
une amorce 1 comprenant une région Seq RC hybride ; et
une deuxième région spécifique qui est une région RC cible contiguë à ladite région d'amorce 1 ; et
une troisième région qui est une région d'amorce 2 contiguë à ladite deuxième région ; et
(ii) l'amplification sélective desdites séquences nucléotidiques cibles ;
dans laquelle amorce 1 fait référence à une amorce comprenant une région de promoteur en 5' pour une ARN polymérase qui catalyse la formation d'un ARN dans le sens 5' → 3' ; Seq RC hybride fait référence à une séquence sur l'extrémité 3' de la séquence de sonde qui est suffisamment complémentaire à l'extrémité 3' de l'amorce 1 pour s'hybrider dans des conditions réactionnelles pour permettre la création d'une séquence d'ADN à double brin par une ADN polymérase ADN-dépendante ; RC cible fait référence à une séquence oligonucléotide courte qui est à complémentarité inversée avec une séquence sur une séquence nucléotidique cible ; et amorce 2 fait référence à une séquence oligonucléotide complémentaire à l'extrémité 3' d'un ARN transcrit à partir de la matrice d'ADN synthétisée dans l'amorce 1, qui s'hybridera à cette séquence pour permettre la transcription d'un hybride ADN/ARN catalysée par une ADN polymérase ARN-dépendante.

2. Procédé selon la revendication 1, comprenant en outre la détection desdites séquences nucléotidiques amplifiées.

3. Procédé selon la revendication 2, dans lequel ladite détection est effectuée par le procédé d'électrophorèse sur gel ou par fluorescence.

4. Procédé selon la revendication 3, dans lequel ladite détection par fluorescence est effectuée par une balise moléculaire.

5. Procédé selon l'une quelconque des revendications 1 à 4, dans lequel ladite amplification de l'étape (ii) comprend la transcription de l'ARN cible hybridé en ADN à double brin.

6. Procédé selon la revendication 5, dans lequel un promoteur contenant une séquence de promoteur de T7 est lié à ladite sonde d'ARN.

7. Procédé selon la revendication 5 ou 6, dans lequel ladite transcription est effectuée au moyen d'une transcriptase inverse, de préférence par l'AMV-RT.

8. Procédé selon l'une quelconque des revendications 5 à 7, comprenant en outre la transcription dudit ADN à double brin résultant en ARN.

9. Procédé selon la revendication 8, dans lequel ladite transcription est effectuée au moyen d'une ARN polymérase qui catalyse la formation d'un ARN dans le sens 5' → 3', la polymérase formant un hybride ADN/ARN à double brin.

10. Procédé selon la revendication 9, dans lequel ladite polymérase est une ARN polymérase de T7.

11. Procédé selon la revendication 9 ou 10, dans lequel seul ledit brin d'ARN d'un hybride ADN/ARN est dégradé.

12. Procédé selon la revendication 11, dans lequel ladite dégradation dudit brin d'ARN est effectuée par une ARNase H.

13. Procédé selon l'une quelconque des revendications 1 à 12, dans lequel ladite région Seq RC hybride, ladite région RC cible et ladite région d'amorce 2 comprennent chacune environ 8 à environ 35 bases, ce nombre pouvant être identique ou différent dans chaque région.

14. Procédé selon l'une quelconque des revendications 1 à 13, comprenant en outre la liaison d'une balise moléculaire à l'ARN amplifié.

Fig. 1

Fig. 2.

Fig. 2A

Fig. 3

Fig. 4

Fig. 5

Fig. 6

Fig. 7

15% DMSO     5% DMSO

Fig. 8

Tris pH 8.0     Tris pH 8.3

Fig. 9

Fig. 10

Fig. 11

Fig. 12

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHAN, P.K.S.** Outbreak of Avian Influenza A(H5N1) Virus Infection in Hong Kong. *Clinical Infectious Diseases,* 1997, vol. 34, 58-S60
- **BRIDGES, C.B. et al.** Prevention and control of influenza: recommendations of the Advisory Committee on Immunization Practices (ACIP). *Morbidity & Mortality Weekly Report,* 2002, vol. 51 (RR-3), 1-31
- **THOMPSON, W.W. et al.** Mortality associated with influenza and respiratory syncytial virus in the United States. *Journal of the American Medical Association,* 2003, vol. 289, 179-86
- **WEBSTER, R.G. et al.** Evolution and Ecology of Influenza A Viruses. *Microbiological Reviews,* 1992, vol. 56 (1), 152-179
- **HINSHAW, V.S. ; R.G. WEBSTER ; B. TURNER.** The perpetuation of orthomyxoviruses and paramyxoviruses in Canadian waterfowl. *Canadian Journal of Microbiology,* 1980, vol. 26, 622-629
- **COX, N.J. ; Z.S. BAI ; A.P. KENDAL.** Laboratory-based surveillance of influenza A (H1N1) and A (H3N2) viruses in 1980-81: antigenic and genomic analysis. *Bulletin of the World Health Organization,* 1983, vol. 61 (1), 143-152
- **WEBSTER, R.G. ; C.H. CAMPBELL ; A. GRANOFF.** The "in vivo" production of "new" influenza A viruses. *Virology,* 1971, vol. 44, 317-328
- **SHORTRIDGE, K.F.** The next pandemic influenza virus?. *The Lancet,* 1995, vol. 346, 1210-12
- **TANG, X. et al.** Isolation and characterization of prevalent strains of avian influenza viruses in China. *Chinese Journal of Animal and Poultry Infectious Diseases,* 1998, vol. 20, 1-5
- **LIGON, B.L.** Avian Influenza Virus H5N1: A Review of Its History and Information Regarding Its Potential to Cause the Next Pandemic. *Seminars in Pediatric Infectious Diseases,* 2005, vol. 16, 326-335
- **WHO.** Avian Influenza: assessing the pandemic threat. *WHO/CDS,* 2005
- **YUEN, K.Y. et al.** Clinical features and rapid viral diagnosis of human disease associated with avian influenza A H5N1 virus. *The Lancet,* 1998, vol. 351, 467-471
- **WHO.** Avian Influenza A (H5N1) Infections in Humans. *The New England Journal of Medicine,* 2005, vol. 353 (13), 1374-85

- **COUCH, R.B.** Prevention and Treatment of Influenza. *The New England Journal of Medicine,* 2000, vol. 343, 1778-1787
- **LA MONTAGNE, J.R. et al.** Summary of clinical trials of inactivated influenza vaccine. *Reviews of Infectious Diseases,* 1983, vol. 5, 723-736
- **PARKMAN, P.D. et al.** Summary of clinical trials of influenza virus vaccines in adults. *Journal of Infectious Diseases,* 1977, vol. 136, 722-S730
- **GROSS, P.A. et al.** The Efficacy of Influenza Vaccine in Elderly Persons: A Meta-Analysis and Review of the Literature. *Annals of Internal Medicine,* 1995, vol. 123 (7), 518-527
- **KROON, F.P. et al.** Antibody response to influenza, tetanus and pneumococcal vaccines in HIV-seropositive indi- viduals in relation to the number of CD4+ lymphocytes. *AIDS,* 1994, vol. 8, 469-476
- **DEMICHELI, V. et al.** Prevention and early treatment of influenza in healthy adults. *Vaccine,* 2000, vol. 18, 957-1030
- **WHO.** Evolution of avian influenza viruses in Asia. *Emerging Infectious Diseases,* 2005, vol. 11, 1515-21
- **STEPHENSON, I. et al.** Development of vaccines against influenza h5. *The Lancet Infectious Diseases,* 2006, vol. 6 (8), 458-460
- **STEPHENSON, I. ; K.G. NICHOLSON.** Influenza: vaccination and treatment. *European Respiratory Journal,* 2001, vol. 17, 1282-1293
- **HAYDEN, F.G.** Antivirals for influenza: Historical perspectives and lessons learned. *Antiviral Research,* 2006, vol. 71, 372-378
- **SABIN, A.B. ; G.G. JACKSON.** Amantadine and Influenza: Evaluation of Conflicting Reports. *The Journal of Infectious Diseases,* 1978, vol. 138 (4), 557-568
- **BRIGHT, R.A. et al.** Adamantane Resistance Among Influenza A Viruses Isolated Early Duping the 2005-2006 Influenza Season in the United States. *The Journal of the American Medical Association,* 2006, vol. 295 (8), 891-894
- **HURT, A.C. et al.** Susceptibility of highly pathogenic A (H5N1) avian influenza viruses to the neuraminidase inhibitors and adamantanes. *Antiviral Research,* 2007, vol. 73, 228-231
- **GUBAREVA, L.V.** Molecular mechanisms of influenza virus resistance to neuraminidase inhibitors. *Virus Research,* 2004, vol. 103, 199-203

- **WOO, P.C.K. et al.** Cost-Effectiveness of Rapid Diagnosis of Viral Respiratory Tract Infections in Pediatric Patients. *Journal of Clinical Microbiology,* 1996, vol. 35 (6), 1579-1581
- **BONNER, A.B. et al.** Impact of the Rapid Diagnosis of Influenza on Physician Decision-Making and Patient Management in the Pediatric Emergence Department: Results of a Randomized, Prospective, Controlled Trial. *Pediatrics,* 2003, vol. 112, 363-367
- **FALSEY, A.R. ; Y. MURATA ; E. E. WALSH.** Impact of Rapid Diagnosis on Management of Adults Hospitalized With Influenza. *Archives of Internal Medicine,* 2007, vol. 167, 354-360
- **ZAMBON, M. et al.** Diagnosis of Influenza in the Community. *Archives of Internal Medicine,* 2001, vol. 161, 2116-2122
- Role of laboratory diagnosis. *CDC,* 08 March 2010, http://www.cdc.gov/flu/professionals/diagnosis/labrole.htm
- **PETRIC, M. ; L. COMANOR ; C. A. PETTI.** Role of the Laboratory in Diagnosis of Influenza during Seasonal Epidemic and Potential Pandemic. *The Journal of Infectious Diseases,* 2006, vol. 194, 98-110
- **FEDORKO, D.P. et al.** Performance of Rapid Tests for Detection of Avian influenza A Virus Types H5N1 and H9N2. *Journal of Clinical Microbiology,* 2006, vol. 44 (4), 1596-1597
- **GAVIN, P.J. ; R. B. THOMSON, JR.** Review of Rapid Diagnostic Tests for Influenza. *Clinical and Applied Immunology Reviews,* 2003, vol. 4, 151-172
- **DEMMLER, G.J.** Laboratory Diagnosis of Influenza: Recent Advances. *Seminars in Pediatric Infectious Diseases,* 2002, vol. 13 (2), 85-90
- **DUNN, J.J. et al.** Comparison of the Denka-Seiken INFLU A.B.-Quick and BD Directigen Flu A+B kits with direct fluorescent antibody staining and shell vial culture methods for rapid detection of influenza viruses. *Journal of Clinical Microbiology,* 2003, vol. 41 (5), 2180-2183
- **SAIKI, R.K. et al.** Enzymatic amplification of beta-globin genomic sequences and restriction site analysis for diagnosis of sickle cell anemia. *Science,* 1985, vol. 230 (4732), 1350-1354
- **SCHOCHETMAN G. ; C. OU ; W. K. JONES.** Polyermase Chain Reaction. *The Journal of Infectious Diseases,* 1988, vol. 158 (6), 1154-1157
- **SAIKI, R.K. et al.** Primer-directed enzymatic amplificaiton of DNA with a thermostable DNA polymerase. *Science,* 1988, vol. 239 (4839), 487-491
- **ELLIS, J.S. ; M. C. ZAMBON.** Molecular diagnosis of influenza. *Reviews in Medical Viorlogy,* 2002, vol. 12, 375-389
- **PAYUNGPORN, S. et al.** Single step multiplex real-time RT-PCR for H5N1 influenza A virus detection. *Journal of Virological Methods,* 2006, vol. 131, 143-147
- **PODDAR, S.K.** Influenza virus types and subtypes detection by single step single tube multiplex reverse transcription-polyermase chain reaction (RT-PCR) and agarose gel electrophoresis. *Journal of Virological Methods,* 2002, vol. 99, 63-70
- **COLLINS, R.A. et al.** Detection of highly pathogenic and low pathogenic avian influenza subtype H5 (Eurasian lineage) using NASBA. *Journal of Virological Methods,* 2002, vol. 103, 213-225
- **GUATELLI, J.C. et al.** Isothermal, in vitro amplification of nucleic acids by a multienzyme reaction modeled after retroviral replication. *Proceedings of the National Academy of Sciences,* 1990, vol. 87, 1874-1878
- **COMPTON, J.** Nucleic acid sequence-based amplification. *Nature,* 1991, vol. 350, 91-92
- **GULLIKSEN, A. et al.** Parallel nanoliter detection of cancer markers using polymer microchips. *The Royal Society of Chemistry,* 2005, vol. 5, 416-420
- **ZAAIJER, H.L. et al.** Detection of HIV-1 RNA in plasma by isothermal amplification (NASBA) irrespective of the stage of HIV-1 infection. *Journal of Virological Methods,* 1995, vol. 52, 175-181
- **LAU, L. et al.** Nucleic acid sequence-based amplification methods to detect avian influenza virus. *Biochemical and Biophysical Research Communications,* 2004, vol. 313, 336-342
- **JEAN, J. ; D.H. D'SOUZA ; L. JAYKUS.** Multiplex Nucleic Acid Sequence-Based Amplification for Simultaneous Detection of Several Enteric Viruses in Model Ready-To-Eat Foods. *Applied and Environmental Microbiology,* 2004, vol. 70 (11), 6603-6610
- **JEAN, J. et al.** Simultaneous detection and identification of hepatitis A virus and rotavirus by multiplex nucleic acid sequence-based amplification (NASBA) and microtiter plate hybridization system. *Journal of Virological Methods,* 2002, vol. 105 (1), 123-132
- **DEIMAN, B. ; P. VAN AARLE ; P. SILLEKENS.** Characteristics and Applications of Nucleic Acid Sequence-Based Amplification (NASBA). *Molecular Biotechnology,* 2002, vol. 20, 163-179
- **MIAO, W.** Electrogenerated Chemiluminescence and Its Biorelated Applications. *Chemical Reviews,* 2008, vol. 108 (7), 2506-2553
- **LEONE, G. et al.** Molecular beacon probes combined with amplification by NASBA enable homogeneous, real-time detection of RNA. *Nucleic Acids Research,* 1998, vol. 26 (9), 2150-2155
- **TYAGI, S. ; F.R. KRAMER.** Molecular Beacons: Probes that Fluoresce upon Hybridization. *Nature Biotechnology,* 1996, vol. 14, 303-308
- **WEUSTEN, J.J.A.M. et al.** Principles of quantitation of viral loads using nucleic acid sequence-based amplification in combination with homogeneous detection using molecular beacons. *Nucleic Acids Research,* 2002, vol. 30 (6), 1-7

- **KERBY, M. et al.** Direct Sequence Detection of Structured H5 Influenza Viral RNA. *The Journal of Molecular Diagnostics,* 2008, vol. 10 (3), 225-235
- **BEHLKE, M.A. et al.** *Fluorescence and Fluorescence Applications,* 2005
- **ARNOLD, S. et al.** Kinetic Modeling and Simulation of In Vitro Transcription by Phage T7 RNA Polymerase. *Biotechnology and Bioengineering,* 2000, vol. 72 (5), 548-561
- **NELSON, D.L. ; M. M. COX.** Lehninger: Principles of Biochemistry. W.H. Freeman and Company, 2008

- **BERGER, S.L. et al.** Reverse Transcriptase and Its Associated Ribonuclease H: Inteiplay of Two Enzyme Activities Controls the Yield of Single-Stranded Complementary Deoxyribonucleic Acid. *Biochemistry,* 1983, vol. 22, 2365-2372
- **MENDELMAN, L.V. ; J. PETRUSKA ; M. F. GOODMAN.** Base Mispair Extension Kinetics. *The Journal of Biological Chemistry,* 1990, vol. 265 (4), 2338-2346
- **GAO, Y. ; L. K. WOLF ; R. M. GEORGIADIS.** Secondary structure effects on DNA hybridization kinetics: a solution versus surface comparison. *Nucleic Acids Research,* 2006, vol. 34 (11), 3370-3377